# EUROPEAN PATENT APPLICATION

(11) **EP 4 782 531 A1**
(43) Date of publication of application: **29.07.2026**
(21) Application number: 24868371.6
(22) Date of filing: 20.09.2024
(51) Int. Cl.: C12M 1/00, C12M 1/26

(54) **CELL DETACHMENT DEVICE AND CELL DETACHMENT METHOD**

(30) Priority: 22.09.2023 JP 2023158473
(71) Applicant: Canon Kabushiki Kaisha, Tokyo 146-8501 (JP); Canon Medical Systems Corporation, Tochigi 324- (JP)
(72) Inventor: IKEDA, Shuhei, Tokyo 146-8501 (JP); EBISAWA, Hisafumi, Tokyo 146-8501 (JP); UCHIDA, Kouichi, Tokyo 146-8501 (JP)
(74) Representative: Marks & Clerk LLP
(86) International application number: PCT/JP2024/033733
(87) International publication number: WO 2025/063295

(57) **Abstract**

A cell detachment apparatus according to embodiments includes a culture vessel and a discharge part. The culture vessel is for culturing cells. The discharge part is provided at a position where the discharge part is not immersed in a liquid in the culture vessel, to detach the cells from a culture surface of the culture vessel by discharging a plurality of liquid droplets toward the culture surface of the culture vessel.

## Description

### FIELD

Embodiments described herein and in the drawings relate generally to a cell detachment apparatus and a cell detachment method.

### BACKGROUND

In the process of culturing cells, cells that have adhered to a bottom surface of a culture vessel need to be detached. For example, a technician manually and physically detaches the cells with a cell scraper or a pipette. However, a manual operation involves risks such as contamination of cells by microorganisms and damage to cells by mechanical stimulation, and therefore a skilled technician needs to perform the detachment operation with extreme caution while ensuring and maintaining a sterile environment.

### CITATION LIST

### PATENT LITERATURE

[PTL 1] Jpn. Pat. Appln. KOKAI Publication No. 2011-155869

### SUMMARY

### TECHNICAL PROBLEM

A problem to be solved by the embodiments disclosed in this specification and drawings is to easily detach cells. However, the problem to be solved by the embodiments disclosed in this specification and the drawings is not limited to the above. Problems corresponding to effects achieved by respective configurations of the embodiments described later can be regarded as other problems.

### SOLUTION TO PROBLEM

A cell detachment apparatus according to an embodiment includes a culture vessel and a discharge part. The culture vessel is configured to culture cells. The discharge part is provided at a position where the discharge part is not immersed in a liquid in the culture vessel, and is configured to detach the cells from a culture surface by discharging a plurality of liquid droplets toward the culture surface of the culture vessel.

### ADVANTAGEOUS EFFECTS OF INVENTION

According to the present invention, cells can be easily detached.

### BRIEF DESCRIPTION OF THE DRAWING(S)

FIG. 1 is a schematic diagram showing a configuration example of a cell detachment apparatus according to a first embodiment.
FIG. 2 is a flowchart showing an operation example of the cell detachment apparatus according to the first embodiment.
FIG. 3 is a schematic diagram showing an operation example of the cell detachment apparatus according to the first embodiment.
FIG. 4 is a diagram showing results of cell detachment under each experimental condition according to the first embodiment.
FIG. 5 is a schematic diagram showing a configuration example of a cell detachment apparatus according to a second embodiment.
FIG. 6 is a flowchart showing an operation example of the cell detachment apparatus according to the second embodiment.
FIG. 7 is a schematic diagram showing an operation example of the cell detachment apparatus according to the second embodiment.

### DETAILED DESCRIPTION

Hereinafter, embodiments will be described with reference to the drawings. In the embodiments described below, parts having the same reference signs are assumed to perform the same operations, and redundant explanations will be omitted as appropriate.

### (First Embodiment)

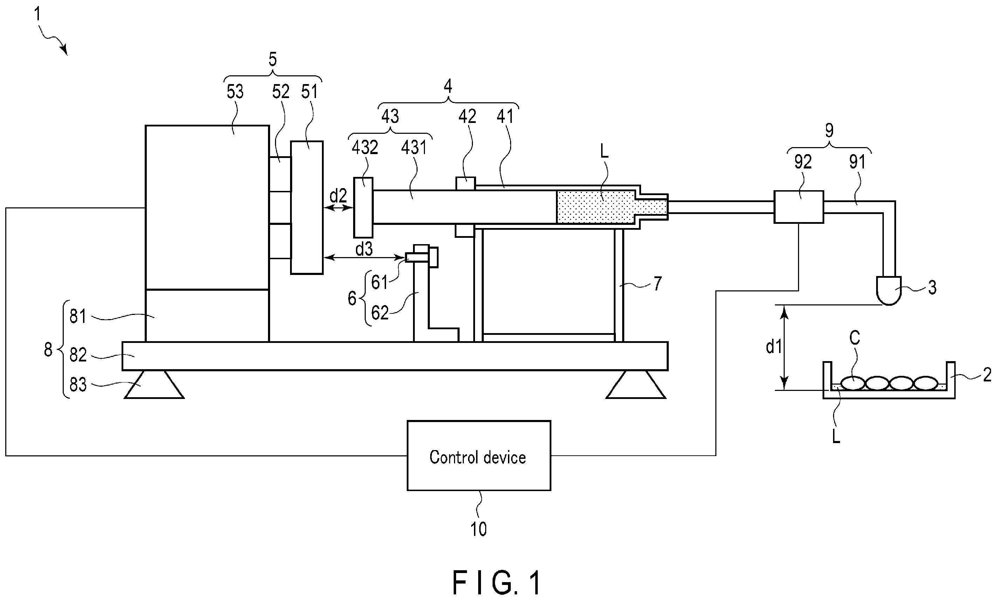

FIG. 1 is a schematic diagram showing a configuration example of a cell detachment apparatus 1 according to a first embodiment. FIG. 1 shows the cell detachment apparatus 1 as viewed from a horizontal direction. The cell detachment apparatus 1 is an apparatus configured to detach cells. The cell detachment apparatus 1 includes a culture vessel 2, a nozzle 3, a syringe 4, a pressing mechanism 5, a stopper 6, a support frame 7, a mounting tool 8, a connector 9, and a control device 10.

The culture vessel 2 is a container configured to culture cells C. The culture vessel 2 is formed of a substance having a property (impermeability) that does not allow a liquid L adhering to a surface to permeate. Examples of such a substance include polyethylene, polypropylene, polycarbonate, polystyrene, and glass. The culture vessel 2 has an arbitrary size and an arbitrary shape. For example, the culture vessel 2 is a circular vessel having a diameter of 35 mm or more and 100 mm or less. The culture vessel 2 is an example of a culture part.

The cells C are cells (adherent cells) that adhere to the surface of the culture vessel 2 via an adhesive protein. Examples of the cells C include induced pluripotent stem (iPS) cells, epithelial cells, endothelial cells, synovial cells, cardiomyocytes, myoblasts, fibroblasts, and neuroblasts. The cells C may be a single cell or a colony (cell cluster) composed of a plurality of cells.

The liquid L is an arbitrary type of liquid. Examples of the liquid L include Phosphate Buffered Saline (PBS) and a liquid medium. The liquid L is preferably a liquid that does not chemically or physiologically damage the cells C.

The nozzle 3 is a mechanical component that discharges the liquid L. The nozzle 3 is formed of a substance such as a metal having relatively high rigidity. The nozzle 3 is physically connected to the syringe 4 by the connector 9. Preferably, a distance d1 between a tip portion of the nozzle 3 and a bottom surface of the culture vessel 2 is 10 mm or more and 50 mm or less. The tip portion of the nozzle 3 is arranged along a straight line that passes through a center of the bottom surface of the culture vessel 2 and is perpendicular to the bottom surface. The bottom surface of the culture vessel 2 functions as a culture surface to which the cells C adhere. The nozzle 3 is arranged above the culture vessel 2, that is, at a position where the nozzle 3 is not immersed in a liquid in the culture vessel 2. The nozzle 3 discharges the liquid L as a plurality of liquid droplets toward the bottom surface of the culture vessel 2. The nozzle 3 is an example of a discharge part.

In the nozzle 3, the number, position, size, and shape of holes (discharge holes) for discharging the liquid L are designed such that conditions (discharge conditions) relating to discharge of the liquid L become desired values. Examples of the discharge conditions include an average particle diameter, an average speed, and an average kinetic energy of the plurality of liquid droplets of the liquid L, and a discharge amount and a discharge time of the liquid L per one discharge. By pressing parameters of the pressing mechanism 5 described later being appropriately set, the nozzle 3 discharges the liquid L in mist form to the cells C cultured in the culture vessel 2 under a desired discharge condition. That is, the nozzle 3 sprays the liquid L onto the cells C to detach the cells C from the culture vessel 2. The liquid droplets of the liquid L discharged from the nozzle 3 fly toward the bottom surface of the culture vessel 2 and collide with the cells C, thus transferring the kinetic energy to the cells C and realizing the detachment of the cells C.

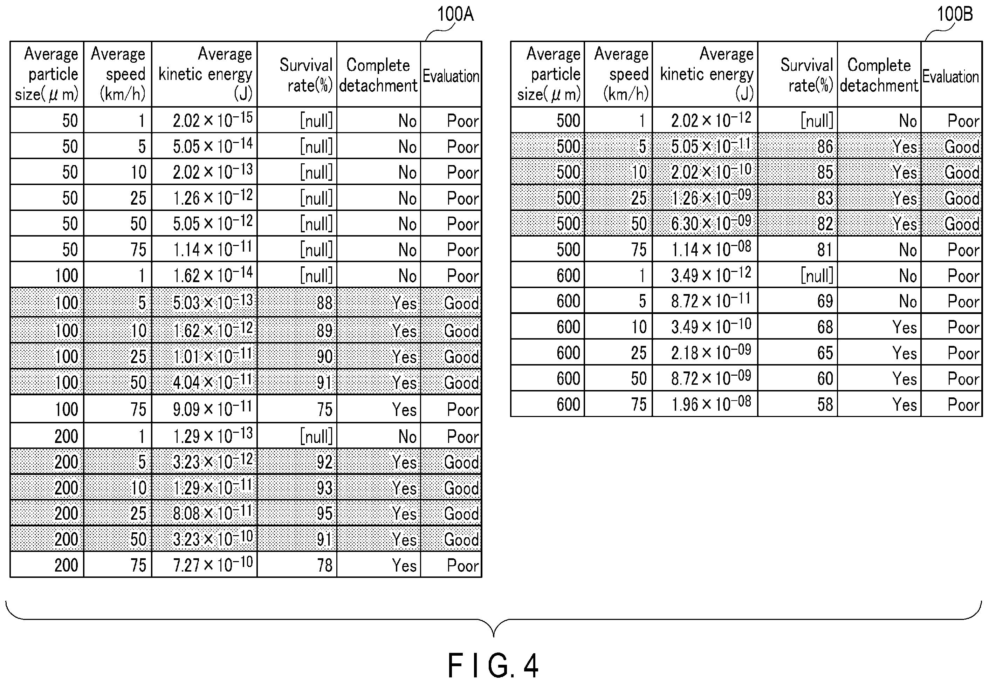

The nozzle 3 discharging the liquid L "in mist form" means that the nozzle 3 discharges the liquid L "as a plurality of minute liquid droplets". The average particle diameter of the plurality of minute liquid droplets is 50 µm or more and 600 µm or less (see FIG. 4). That is, "mist form" does not refer to the shape (for example, a conical shape or a linear shape) of a spatial range in which the liquid droplets are discharged, but indicates a state related to the size of the liquid droplets.

The nozzle 3 may detach the cells C from the culture vessel 2 by discharging the liquid L once. At this time, the discharge amount of the liquid L per one discharge, that is, a total amount of the liquid droplets discharged in one discharge is preferably 3.0 mL or less. The time for one discharge of the liquid L is preferably 0.2 seconds or less. A discharge angle of the liquid L from the nozzle 3 may be designed or adjusted so that the discharged liquid L contacts an entire surface (preferably, an area of 95% or more) of the cells C. Typically, the nozzle 3 discharges the liquid L in a conical shape. The nozzle 3 may discharge the liquid L linearly.

The nozzle 3 may discharge the liquid L to a portion of the surface of the cells C cultured in the culture vessel 2 that is in contact with air. That is, the nozzle 3 discharges the liquid L not to a portion of the surface of the cells C that is immersed in the liquid L (immersed portion) but to the portion of the surface of the cells C that is in contact with air. The nozzle 3 directly transmits the kinetic energy of the discharged liquid L to the cells C without the liquid L covering the surfaces of the cells C, and thus the cells C can be effectively detached from the culture vessel 2.

The syringe 4 is a device configured to store the liquid L and transmit kinetic energy to the liquid L. The syringe 4 is formed of, for example, a substance similar to that of the culture vessel 2. The syringe 4 is arranged in the horizontal direction (a left-right direction in FIG. 1). The syringe 4 has a barrel 41, a flange 42, and a plunger 43.

The barrel 41 is a stationary component configured to store the liquid L. The barrel 41 is a hollow cylindrical container. One end part of the barrel 41 is connected to the nozzle 3 by a connector 9. The other end part of the barrel 41 is connected to the plunger 43 by the flange 42. A lower portion of the barrel 41 is physically supported by the support frame 7. The liquid L may be stored in the barrel 41 in advance by a user, or the liquid L may be automatically stored by a known configuration. The liquid L may be repeatedly stored in the barrel 41. The barrel 41 is an example of a reservoir.

The flange 42 is a stationary component configured to couple the barrel 41 and the plunger 43. The flange 42 couples the barrel 41 and the plunger 43 such that the plunger 43 is movable along a longitudinal direction of the barrel 41 (a left-right direction in FIG. 1). The flange 42 is an example of a coupling part.

The plunger 43 is an operating component configured to transmit kinetic energy to the liquid L. The plunger 43 includes a rod 431 and a pressure receiving plate 432. The rod 431 and the pressure receiving plate 432 may be integrally formed. The plunger 43 is an example of a transmission part.

The rod 431 is a bar configured to cause the liquid L stored in the barrel 41 to move. The rod 431 is a cylinder having a diameter corresponding to an inner diameter of the barrel 41. One end part of the rod 431 is mounted inside the barrel 41. The other end part of the rod 431 is coupled to the pressure receiving plate 432. The rod 431 moves in the same direction as the movement direction of the pressure receiving plate 432, thus pushing out the liquid L toward the nozzle 3. The rod 431 directly transfers kinetic energy to the liquid L.

The pressure receiving plate 432 is a plate that receives pressure from the pressing mechanism 5. The pressure receiving plate 432 moves in a direction in which the pressure is received, thereby moving the rod 431 in the same direction.

The pressing mechanism 5 is a mechanism that presses the pressure receiving plate 432. The pressing mechanism 5 is formed of a substance such as a metal having a relatively high rigidity. The pressing mechanism 5 is electrically connected to the control device 10 by an electric wire. The pressing mechanism 5 adjusts parameters related to the pressing of the pressure receiving plate 432 (pressing parameters) so as to realize a desired discharge condition according to the control by the control device 10. Examples of the pressing parameters include a pressing force and a pressing time. The pressing mechanism 5 presses the pressure receiving plate 432 with the adjusted pressing parameters. The pressing mechanism 5 includes a pressing plate 51, an arm part 52, and a base 53. The pressing mechanism 5 is an example of a pressing portion.

The pressing plate 51 is an operating component that presses the pressure receiving plate 432. One surface of the pressing plate 51 faces one surface of the pressure receiving plate 432. A distance d2 between one of the surfaces of the pressing plate 51 and one of the surfaces of the pressure receiving plate 432 is designed as an arbitrary value. The other surface of the pressing plate 51 is coupled to the arm part 52.

The arm part 52 is an operating component that moves the pressing plate 51. The arm part 52 moves the pressing plate 51 in a same direction by expanding and contracting along the longitudinal direction of the barrel 41.

The base 53 is a stationary component that physically supports the arm part 52. The base 53 houses a part of the arm part 52. The base 53 is fixed to an upper surface of the mounting tool 8.

The stopper 6 is a device that stops the movement of the pressing plate 51. The stopper 6 is arranged between the pressing plate 51 and the flange 42. The stopper 6 includes an absorbing member 61 and a supporting member 62. The stopper 6 is an example of a stopping part.

The absorbing member 61 is a stationary component that absorbs pressure from the pressing plate 51. The absorbing member 61 is formed of a substance having a cushioning property. Examples of such a substance include urethanes, rubbers, and gels. A distance d3 between the absorbing member 61 and the surface of the pressing plate 51 is designed to be a value greater than the distance d2 (d3 > d2). A distance obtained by subtracting the distance d2 from the distance d3 (d3 - d2) corresponds to the distance that the plunger 43 can move.

The supporting member 62 is a stationary component configured to physically support the absorbing member 61. The supporting member 62 is formed of a substance such as a metal having a relatively high rigidity. The supporting member 62 has an L-shape, and includes a first portion extending in a vertical direction (an up-down direction in FIG. 1) and a second portion extending in the horizontal direction. A height of the first portion is designed such that the first portion does not interfere with the movement of the plunger 43 and stops the movement of the pressing plate 51. The absorbing member 61 is arranged at an upper portion of the first portion. The second portion is fixed to the upper surface of the mounting tool 8.

The support frame 7 is a stationary component that physically supports the barrel 41. The support frame 7 adjusts the height at which the barrel 41 is placed. The support frame 7 is formed of a substance such as a metal having a relatively high rigidity. The support frame 7 is fixed to the upper surface of the mounting tool 8. The support frame 7 is an example of a support part.

The mounting tool 8 is a device on which the pressing mechanism 5, the stopper 6, and the support frame 7 are mounted. The mounting tool 8 is formed of a substance such as a metal having a relatively high rigidity. The mounting tool 8 has a pedestal 81, a plate 82, and leg parts 83. The mounting tool 8 is an example of a mounting part.

The pedestal 81 is a stationary component on which the base 53 is placed. The pedestal 81 is configured to adjust the height at which the base 53 is placed. The height of the pedestal 81 is designed such that the pressing plate 51 can press the pressure receiving plate 432. The pedestal 81 is fixed to the upper surface of the plate 82.

The plate 82 is a stationary component on which the stopper 6 and the support frame 7 are mounted. One end part and the other end part of the plate 82 are coupled to the leg parts 83.

The leg parts 83 are stationary components that adjust the height of the plate 82 from a floor surface. The leg parts 83 are placed on the floor surface. A user of the cell detachment apparatus 1 can insert their fingers into a gap formed between the plate 82 and the floor surface and lift up the plate 82 from the floor surface.

The connector 9 is a device configured to connect the nozzle 3 and the barrel 41 to each other. The connector 9 includes a supply tube 91 and a flow rate sensor 92. The connector 9 is an example of a connecting part.

The supply tube 91 is a tube configured to supply the liquid L sent from the barrel 41 to the nozzle 3. The supply tube 91 is formed of a silicone rubber, polyethylene, a metal, or the like. A flow rate sensor 92 may be arranged in a part of the supply tube 91.

The flow rate sensor 92 is a sensor configured to measure a flow rate of the liquid L passing through the supply tube 91. The flow rate sensor 92 is electrically connected to the control device 10 by an electric wire. The flow rate sensor 92 measures the flow rate of the liquid L under the control of the control device 10, and transmits a measurement value of the flow rate to the control device 10. The control device 10 may determine set values of the pressing parameters based on the relationship between the set pressing parameters and the flow rate measured by the flow rate sensor 92. For example, the control device 10 calculates a conversion formula between the pressing parameters and the flow rates by measuring each flow rate for the discharge operations based on the plurality of pressing parameters. The control device 10 may calculate pressing parameters corresponding to a flow rate that realizes a desired discharge condition based on the conversion formula, and may determine the calculated pressing parameters as new set values.

The control device 10 is a device configured to control the entire operation of the cell detachment apparatus 1. The control device 10 includes at least one processor. The processor refers to, for example, circuitry such as a central processing unit (CPU), a graphics processing unit (GPU), an application specific integrated circuit (ASIC), a programmable logic device (for example, a simple programmable logic device (SPLD), a complex programmable logic device (CPLD), or a field programmable gate array (FPGA)), etc. If the processor is a CPU, the CPU reads and executes a control program stored in a memory to realize each function. If the processor is an ASIC, each function is directly incorporated as a logic circuit into circuitry of the ASIC. The processor may be configured as a single circuitry or may be configured as a combination of a plurality of independent circuitry. The control device 10 is an example of a controller.

The control device 10 may be an information processing device such as a general-purpose computer. The control device 10 may have each component included in a general-purpose computer (for example, a processor, a memory, an input device, an output device, and a communication device). The control device 10 may receive various instructions (for example, a discharge instruction, a drain instruction, and an end instruction) from the user via the input device. The control device 10 may present the measurement value of the flow rate from the flow rate sensor 92 to the user in a predetermined mode by the output device.

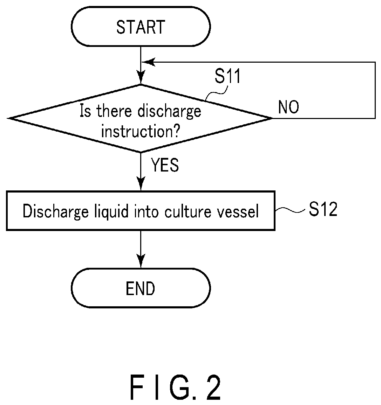

FIG. 2 is a flowchart showing an operation example of the cell detachment apparatus 1 according to the first embodiment. According to the present example, the cell detachment apparatus 1 discharges the liquid L to the culture vessel 2 in accordance with a discharge instruction from the user.

(Step S11) First, the cell detachment apparatus 1 determines whether or not a discharge instruction has been received from the user. For example, the control device 10 determines whether or not the user has input the discharge instruction to the control device 10 using an input device (for example, a mouse, a keyboard, or a communication terminal). In a case where the discharge instruction is input (step S11: YES), the process proceeds to step S12. In a case where the discharge instruction is not input (step S11: NO), the process returns to step S11. That is, the control device 10 is in a standby state until the discharge instruction is received from the user. The discharge instruction may include a discharge condition related to the discharge of the liquid L.

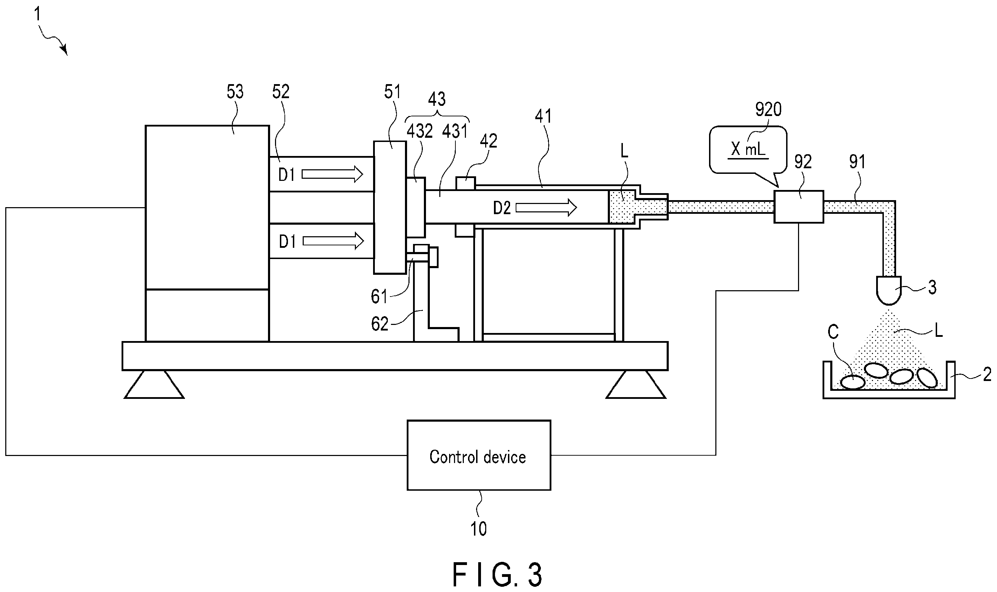

(Step S12) Finally, the cell detachment apparatus 1 discharges the liquid L into the culture vessel 2. Specifically, the control device 10 controls the pressing mechanism 5 to cause the pressing plate 51 to press the pressure receiving plate 432, and discharge the liquid L from the nozzle 3 to the culture vessel 2 under a desired discharge condition (see FIG. 3).

FIG. 3 is a schematic diagram showing an operation example of the cell detachment apparatus 1 according to the first embodiment. As shown in FIG. 3, the control device 10 extends the arm part 52 of the pressing mechanism 5 along a direction D1, thus making the pressing plate 51 move along the direction D1. The pressing plate 51 comes in contact with the pressure receiving plate 432 and presses the pressure receiving plate 432 along a direction D2 which is the same direction as the direction D1. The pressure receiving plate 432 moves the rod 431 in the direction D2, thus pushing out the liquid L stored in the barrel 41 along the direction D2. The movement of the pressing plate 51 is stopped by coming into contact with the absorbing member 61.

The liquid L pushed out from the barrel 41 passes through the supply tube 91 and reaches the nozzle 3. The nozzle 3 discharges the supplied liquid L to the culture vessel 2 under a desired discharge condition. The nozzle 3 sprays the liquid L to the culture vessel 2 to thus detach the cells C from the culture vessel 2. In particular, the cells C detached by the discharge of the liquid L are in a state of being separated into individual cells (single cells). The flow rate sensor 92 measures the flow rate of the liquid L passing through the supply tube 91 and transmits a measurement value 920 of the flow rate to the control device 10.

FIG. 4 is a diagram illustrating cell detachment results for each experimental condition according to the first embodiment. Tables 100A and 100B show detachment results of the cells C obtained by the nozzle 3 discharging the liquid L to the cells C under the respective values of the three types of discharge conditions (average particle size, average speed, and average kinetic energy). The following illustrates the experimental procedures performed to obtain the present detachment results.

As the cells C, human-derived iPS cells (particularly, P0 cells) were used. As the culture vessel 2, a circular vessel (35 mm dish) having a 35 mm diameter was used. First, the experimenter added iPS cells and a liquid medium to the circular vessel, allowed the iPS cells to form a colony, and then sucked out all the liquid medium. The experimenter then washed the colony by adding 1 mL of the PBS to the circular vessel and sucking out all of the PBS. Subsequently, the experimenter added 400 µL of ethylenediaminetetraacetic acid (EDTA) to the circular vessel, incubated the colony at 37 °C for 10 minutes, and then sucked out all of the EDTA. Next, the experimenter washed the colony again by adding the PBS to the circular vessel and sucking out all of the PBS.

Subsequently, the experimenter sprayed 1 mL of the PBS from the nozzle 3 to the colony at each of values according to the three types of discharge conditions (average particle size, average speed, and average kinetic energy), thus detaching the colony from the circular vessel. The experimenter measured a "survival rate" of the detached colony. Furthermore, the experimenter visually observed the state of detachment of the colony from the circular vessel with a microscope. Based on the observation results, the experimenter determined whether or not there was "complete detachment" of the detached colony and the quality of the "evaluation".

Specifically, the "survival rate" was measured by the following method. First, the experimenter arbitrarily took 10 µL out of 1 mL of cell suspension after detaching, and mixed the 10 µL cell suspension with 10 µL of trypan blue to obtain a mixed solution. The experimenter then injected the mixed solution onto an automated cell-counter slide, inserted the slide into an automated cell-counter (BioRad, TC20 (registered trademark)), and measured the number of cells and the survival rate using multifocal plane analysis.

In a case where the colony was completely detached from the bottom surface of the circular vessel, the experimenter determined "complete detachment: YES". In a case where the "survival rate" of the cells after the detachment was 80% or more and "complete detachment: YES", then the experimenter determined "evaluation: good".

In Tables 100A and 100B, each of the records relating to "evaluation: good" is highlighted. According to the highlighted records, the average particle size is 100 µm or more and 500 µm or less, the average speed is 5 km/h or more and 50 km/h or less, and the average kinetic energy is 5.0 × 10⁻¹³ J or more and 6.3 × 10⁻⁹ J or less. That is, in a case where the three types of discharge conditions satisfy the above conditions, the colony of the iPS cells can be particularly effectively detached from the circular vessel.

In a case where the PBS was sprayed on the colony in a state where the PBS remained in the circular vessel (that is, the colony of the iPS cells was immersed in the PBS), a ratio of an area detached from the circular vessel to the area of the entire colony (detached area ratio) decreased. (i) In a case where no PBS remained, the detached area ratio was "98%". (ii) In a case where 400 µL of the PBS remained, the detached area ratio was "45%". (iii) In a case where 1 mL of the PBS remained, the detached area ratio was "5%". That is, it is desirable to expand the portion of the surface of the colony that is in contact with air by sufficiently draining the PBS from the circular vessel prior to the detachment of the colony.

In other words, in the cell detachment apparatus 1 according to the first embodiment, prior to the step (S12) of discharging the liquid L to the culture vessel 2, the liquid in the culture vessel 2 may be drained through a liquid port not shown, by driving a liquid pump not shown, to reduce an amount of the liquid in the culture vessel 2. In this case, as the liquid pump and the liquid port, for example, similar ones as a liquid pump 25B and a liquid port 211B in a second embodiment to be described later can be respectively used.

According to the first embodiment described above, the cell detachment apparatus 1 detaches the cells C cultured in the culture vessel 2 by discharging the liquid L from the nozzle 3 to the cells C. Accordingly, the user can easily detach the cells C without requiring complicated manual work. The cell detachment apparatus 1 has the nozzle 3 at a position where the nozzle is not immersed in the liquid L in the culture vessel 2. Accordingly, the user can effectively detach the cells C using the action of collision of the liquid droplets. Further, the cell detachment apparatus 1 ejects the liquid L from the fixed nozzle 3 to detach the cells C. Therefore, the configuration of the cell detachment apparatus 1 can be simplified.

In addition, the cell detachment apparatus 1 directly transmits the kinetic energy of the discharged liquid L to the cells C without passing through the wall surface of the culture vessel 2. Therefore, the cell detachment apparatus 1 can effectively detach the cells C. In particular, the cell detachment apparatus 1 can separate a colony of the cells C into individual cells C (single cell formation).

### (Second Embodiment)

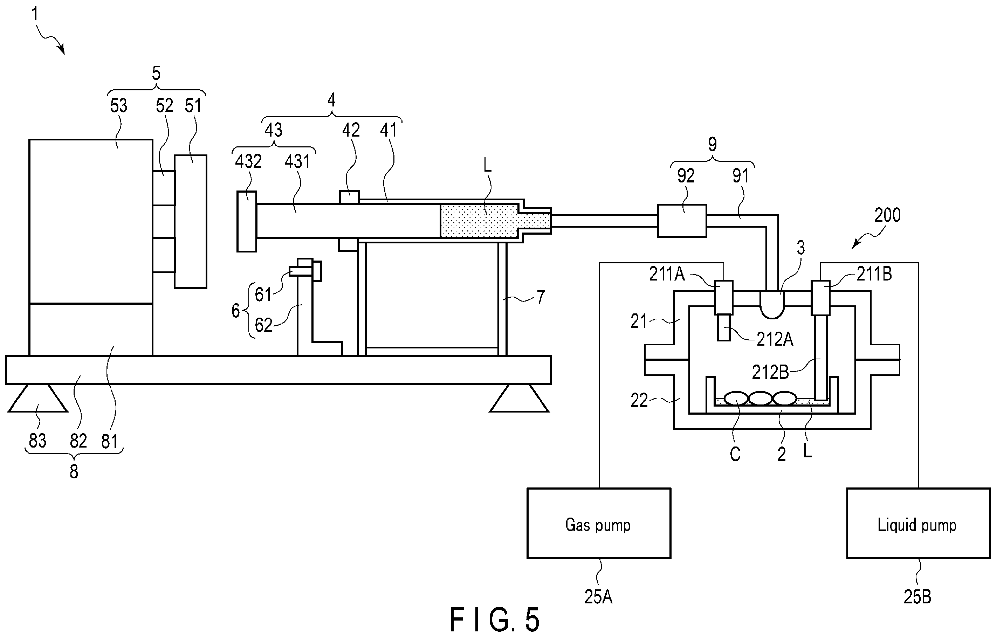

FIG. 5 is a schematic diagram showing a configuration example of a cell detachment apparatus 1 according to a second embodiment. The cell detachment apparatus 1 according to the second embodiment includes a cell detachment vessel 200, a gas pump 25A, and a liquid pump 25B in addition to the components according to the first embodiment (see FIG. 1). The gas pump 25A and the liquid pump 25B may be electrically connected to the control device 10 by electric wires.

The cell detachment vessel 200 is a container configured to detach cells. The cell detachment vessel 200 is a closed-system container. The cell detachment vessel 200 includes an upper lid 21, a lower lid 22, a gas port 211A, a liquid port 211B, a gas tube 212A, and a liquid tube 212B.

The upper lid 21 and the lower lid 22 are lids configured to form a housing of the cell detachment vessel 200. The upper lid 21 and the lower lid 22 are formed of a substance such as a metal having a relatively high rigidity. The upper lid 21 and the lower lid 22 have a U-shaped cross section, and have a flat bottom surface, and L-shaped side surfaces. The upper lid 21 and the lower lid 22 are coupled to each other in a state where the side surfaces of the upper lid 21 and the side surfaces of the lower lid 22 are in contact with each other. By this coupling, the inside of the cell detachment vessel 200 is physically blocked from the outside and is kept airtight. The upper lid 21 and the lower lid 22 may be detachably coupled to each other.

The gas port 211A, the liquid port 211B, and the nozzle 3 are arranged on the bottom surface of the upper lid 21 so as to penetrate the bottom surface. The nozzle 3 is arranged in the center of the bottom surface. The nozzle 3 may be detachably arranged at the bottom surface. The culture vessel 2 is placed on the bottom surface of the lower lid 22. The bottom surface of the lower lid 22 may be placed on the floor surface.

The gas port 211A and the liquid port 211B are ports configured to exchange a fluid between the inside and the outside of the cell detachment vessel 200. The gas port 211A and the liquid port 211B are formed of substances such as metals, resins, and plastics. The gas tube 212A is connected to one end part of the gas port 211A. The gas pump 25A is connected to the other end part of the gas port 211A by a tube. The liquid tube 212B is connected to one end part of the liquid port 211B. The liquid pump 25B is connected to the other end part of the liquid port 211B by a tube.

The gas tube 212A and the liquid tube 212B are tubes configured to exchange fluid between the inside and the outside of the cell detachment vessel 200. The gas tube 212A and the liquid tube 212B are formed of, for example, a similar substance as that of the supply tube 91. The length of the gas tube 212A is designed such that a tip end part of the gas tube 212A does not come into contact with the liquid L discharged from the nozzle 3. The length of the liquid tube 212B is designed such that a tip end part of the liquid tube 212B nearly contacts the bottom surface of the culture vessel 2.

The gas pump 25A and the liquid pump 25B are pumps that exchange a fluid between the inside and the outside of the cell detachment vessel 200. The gas pump 25A and the liquid pump 25B may send the fluid to the inside of the cell detachment vessel 200 or may drain the fluid from the inside of the cell detachment vessel 200 according to control by the control device 10. The gas pump 25A sends or drains an arbitrary type of gas. The liquid pump 25B sends or drains an arbitrary type of liquid. The liquid pump 25B is an example of a sending part and a drain part. A liquid pump other than the liquid pump 25B may be added, and one of the two liquid pumps may be used for sending the liquid and the other may be used for draining the liquid.

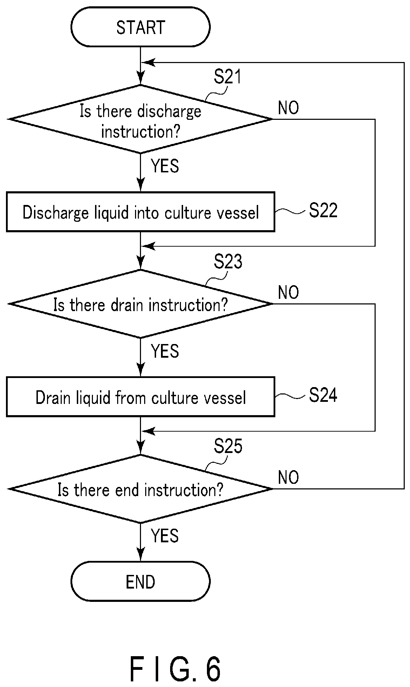

FIG. 6 is a flowchart showing an operation example of the cell detachment apparatus 1 according to the second embodiment. The cell detachment apparatus 1 according to the second embodiment drains the liquid L from the culture vessel 2 according to a discharge instruction from the user in addition to the same operations as that of the first embodiment (see FIG. 2).

(Step S21) First, the cell detachment apparatus 1 determines whether or not a discharge instruction has been received from the user. Step S21 is similar to step S11. In a case where the discharge instruction is input (step S21: YES), the process proceeds to step S22. In a case where the discharge instruction is not input (step S21: NO), the process proceeds to step S23.

(Step S22) Next, the cell detachment apparatus 1 discharges the liquid L to the culture vessel 2. Step S22 is similar to step S12.

(Step S23) Subsequently, the cell detachment apparatus 1 determines whether or not a drain instruction has been received from the user. For example, the control device 10 determines whether or not the user has input the drain instruction to the control device 10 using an input device. In a case where the drain instruction is input (step S23: YES), the process proceeds to step S24. In a case where the drain instruction has not been input (step S23: NO), the process proceeds to step S25.

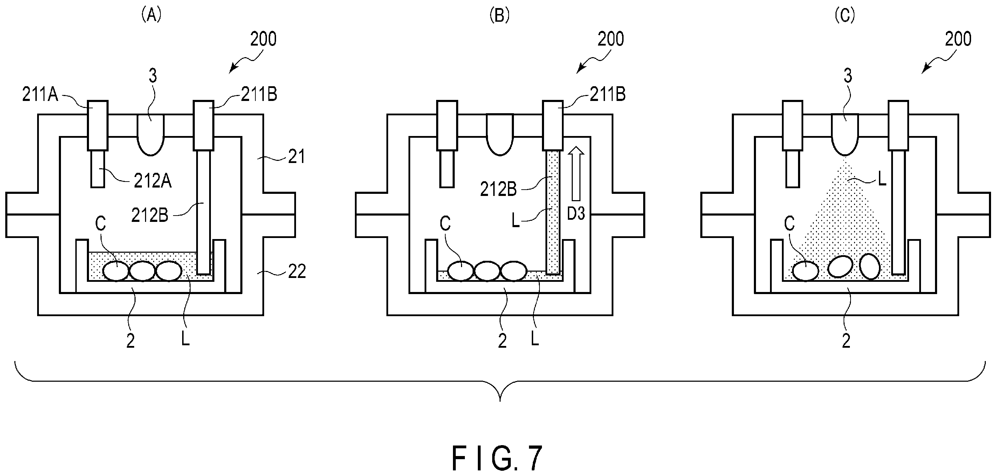

(Step S24) Subsequently, the cell detachment apparatus 1 drains the liquid L from the culture vessel 2. Specifically, the control device 10 controls the liquid pump 25B to suck out the liquid L in the culture vessel 2 by the liquid pump 25B. The liquid pump 25B drains the liquid L from the culture vessel 2 through the liquid port 211B and the liquid tube 212B (see FIG. 7).

(Step S25) Finally, the cell detachment apparatus 1 determines whether or not an end instruction has been received from the user. For example, the control device 10 determines whether or not the user has input an end instruction to the control device 10 using the input device. In a case where the end instruction is input (step S25: YES), the cell detachment apparatus 1 ends the series of operations. In a case where the end instruction is not input (step S25: NO), the process returns to step S21. That is, the control device 10 repeats the processes from step S21 to step S24 until the control device 10 receives an end instruction from the user.

FIG. 7 is a schematic diagram showing an operation example of the cell detachment apparatus 1 according to the second embodiment. FIG. 7 shows the cell detachment vessel 200 in each state in chronological order.

As shown in FIG. 7(A), the liquid L remains in the culture vessel 2. The cells C cultured in the culture vessel 2 are immersed in the liquid L. At this time, at least a part of the surface of the colony of the cells C may be immersed in the liquid L.

As shown in FIG. 7(B), the liquid pump 25B drains the liquid L from the culture vessel 2 through the liquid port 211B and the liquid tube 212B. The liquid pump 25B drains the liquid L vertically upward along a direction D3. By draining the liquid L, the amount of the liquid L in the culture vessel 2 decreases, and a portion of the surface of the cells C that is in contact with air appears and expands.

As shown in FIG. 7(C), the nozzle 3 sprays the liquid L onto the cells C in a state where the liquid L hardly remains in the culture vessel 2. That is, the nozzle 3 sprays the liquid L onto the portion of the cells C that is in contact with air, which is enlarged by draining of the liquid L, and thus the cells C can be effectively detached from the culture vessel 2.

According to the second embodiment described above, the cell detachment apparatus 1 drains the liquid L remaining in the culture vessel 2 by the liquid pump 25B. The cell detachment apparatus 1 discharges the liquid L from the nozzle 3 in a state where the liquid L hardly remains or does not remain in the culture vessel 2. The cell detachment apparatus 1 directly transmits the kinetic energy of the discharged liquid L to the cells C without the liquid L covering the surfaces of the cells C, and thus can effectively detach the cells C from the culture vessel 2.

Further, the cell detachment apparatus 1 can detach the cells C aseptically and automatically using the cell detachment vessel 200 held in an airtight manner. The cell detachment apparatus 1 exchanges gas by the gas pump 25A and exchanges liquid by the liquid pump 25B between the inside and the outside of the cell detachment vessel 200. Therefore, the cell detachment apparatus 1 can aseptically and automatically culture the cells C inside the cell detachment vessel 200. From this viewpoint, the cell detachment apparatus 1 is also referred to as a "cell culture apparatus" or a "cell production apparatus".

According to at least one of the embodiments described above, the cells can be easily detached.

While several embodiments have been described, these embodiments have been presented by way of example only, and are not intended to limit the scope of the inventions. These embodiments can be implemented in other various forms, and various omissions, replacements, changes, and combinations of the embodiments can be made without departing from the spirit of the invention. These embodiments and their modifications are included in the scope and spirit of the invention, and are also included in the scope of the claimed inventions and their equivalents.

With respect to the above embodiments, the following additional notes are disclosed as aspects and optional features of the inventions.

### (Additional Note 1)

A cell detachment apparatus, comprising: a culture vessel for culturing cells; and
a discharge part provided at a position where the discharge part is not immersed in a liquid in the culture vessel, and configured to detach the cells from a culture surface of the culture vessel by discharging a plurality of liquid droplets toward the culture surface of the culture vessel.

### (Additional Note 2)

The discharge part may be configured to discharge the plurality of liquid droplets to a portion of a surface of the cells that is in contact with air.

### (Additional Note 3)

An average particle diameter of the plurality of liquid droplets may be 100 µm or more and 500 µm or less.

### (Additional Note 4)

An average speed of the plurality of liquid droplets may be 5 km/h or more and 50 km/h or less.

### (Additional Note 5)

An average kinetic energy of the plurality of liquid droplets may be 5.0 × 10⁻¹³ J or more and 6.3 × 10⁻⁹ J or less.

### (Additional Note 6)

A total discharge amount of the plurality of liquid droplets by one discharge may be 3.0 mL or less.

### (Additional Note 7)

A discharge time of the plurality of liquid droplets by one discharge may be 0.2 seconds or less.

### (Additional Note 8)

A distance between the culture surface and the discharge part may be 10 mm or more and 50 mm or less.

### (Additional Note 9)

The cell detachment apparatus may further comprise a reservoir connected to the discharge part and configured to store a liquid.

The cell detachment apparatus may further comprise a transmission part configured to transmit kinetic energy to the liquid stored in the reservoir.

The liquid stored in the reservoir may be discharged by the discharge part as the plurality of liquid droplets in a state where the kinetic energy is transmitted by the transmission part.

### (Additional Note 10)

The reservoir may be a hollow and cylindrical barrel including one end part connected to the discharge part.

The transmission part may be a plunger mounted inside the barrel and configured to cause the liquid to move toward the one end part.

### (Additional Note 11)

The cell detachment apparatus may further comprise a drain part for draining a liquid from the culture vessel.

The drain part may be configured to drain a liquid from the culture vessel to reduce an amount of a liquid in the culture vessel so that a portion of the surface of the cells that is in contact with air is expanded and the discharge part may discharge the plurality of liquid droplets to the expanded portion in contact with air.

### (Additional Note 12)

A cell detachment method, comprising: detaching cells cultured in a culture vessel from a culture surface of the culture vessel by discharging a plurality of liquid droplets toward the culture surface of the culture vessel from a position where a discharge part is not immersed in a liquid in the culture vessel.

### (Additional Note 13)

In the cell detachment method, the plurality of liquid droplets may be discharged to a portion of a surface of the cells that is in contact with air.

### (Additional Note 14)

An average particle diameter of the plurality of liquid droplets may be 100 µm or more and 500 µm or less.

### (Additional Note 15)

An average speed of the plurality of liquid droplets may be 5 km/h or more and 50 km/h or less.

### (Additional Note 16)

An average kinetic energy of the plurality of liquid droplets may be 5.0 × 10⁻¹³ J or more and 6.3 × 10⁻⁹ J or less.

### (Additional Note 17)

A total discharge amount of the plurality of liquid droplets by one discharge may be 3.0 mL or less.

### (Additional Note 18)

A discharge time of the plurality of liquid droplets by one discharge may be 0.2 seconds or less.

### (Additional Note 19)

A distance between the culture surface and the position may be 10 mm or more and 50 mm or less.

### (Additional Note 20)

The cell detachment method includes draining the liquid from the culture vessel to reduce an amount of the liquid in the culture vessel so that a portion of the surface of the cells that is in contact with air is expanded, and the plurality of liquid droplets may be discharged to the expanded portion in contact with air.

### REFERENCE SIGNS LIST

1 cell detachment apparatus, 2 culture vessel, 3 nozzle, 4 syringe, 5 pressing mechanism, 6 stopper, 7 support frame, 8 mounting tool, 9 connector, 10 control device, 21 upper lid, 22 lower lid, 25A gas pump, 25B liquid pump, 41 barrel, 42 flange, 43 plunger, 51 pressing plate, 52 arm part, 53 base, 61 absorbing member, 62 supporting member, 81 pedestal, 82 plate, 83 leg part, 91 supply tube, 92 flow sensor, 100A, 100B table, 200 cell detachment vessel, 211A gas port, 211B liquid port, 212A gas tube, 212B liquid tube, 431 rod, 432 pressure receiving plate, 920 measurement value, C cell, d1, d2, d3 distance, D1, D2, D3 direction, L liquid

## Claims

1. A cell detachment apparatus, comprising:
a culture vessel for culturing cells; and
a discharge part provided at a position where the discharge part is not immersed in a liquid in the culture vessel, and configured to detach the cells from a culture surface of the culture vessel by discharging a plurality of liquid droplets toward the culture surface of the culture vessel.

2. The cell detachment apparatus according to claim 1, wherein the discharge part is configured to discharge the plurality of liquid droplets to a portion of a surface of the cells that is in contact with air.

3. The cell detachment apparatus according to claim 1 or 2, wherein an average particle diameter of the plurality of liquid droplets is 100 µm or more and 500 µm or less.

4. The cell detachment apparatus according to any one of claims 1 to 3, wherein an average speed of the plurality of liquid droplets is 5 km/h or more and 50 km/h or less.

5. The cell detachment apparatus according to any one of claims 1 to 4, wherein an average kinetic energy of the plurality of liquid droplets is 5.0 × 10⁻¹³ J or more and 6.3 × 10⁻⁹ J or less.

6. The cell detachment apparatus according to any one of claims 1 to 5, wherein a total discharge amount of the plurality of liquid droplets by one discharge is 3.0 mL or less.

7. The cell detachment apparatus according to any one of claims 1 to 6, wherein a discharge time of the plurality of liquid droplets by one discharge is 0.2 seconds or less.

8. The cell detachment apparatus according to any one of claims 1 to 7, wherein a distance between the culture surface and the discharge part is 10 mm or more and 50 mm or less.

9. The cell detachment apparatus according to any one of claims 1 to 8, further comprising:
a reservoir connected to the discharge part and configured to store a liquid; and
a transmission part configured to transmit kinetic energy to the liquid stored in the reservoir,
wherein the liquid stored in the reservoir is discharged by the discharge part as the plurality of liquid droplets in a state where the kinetic energy is transmitted by the transmission part.

10. The cell detachment apparatus according to claim 9, wherein
the reservoir is a hollow and cylindrical barrel including one end part connected to the discharge part, and
the transmission part is a plunger mounted inside the barrel and configured to cause the liquid to move toward the one end part.

11. The cell detachment apparatus according to any one of claims 1 to 10, further comprising:
a drain part for draining a liquid from the culture vessel,
wherein the drain part is configured to drain a liquid from the culture vessel to reduce an amount of a liquid in the culture vessel so that a portion of the surface of the cells that is in contact with air is expanded and the discharge part discharges the plurality of liquid droplets to the expanded portion in contact with air.

12. A cell detachment method comprising:
detaching cells cultured in a culture vessel from a culture surface of the culture vessel by discharging a plurality of liquid droplets toward the culture surface of the culture vessel from a position where a discharge part is not immersed in a liquid in the culture vessel.

13. The cell detachment method according to claim 12, wherein the plurality of liquid droplets are discharged to a portion of a surface of the cells that is in contact with air.

14. The cell detachment method according to claim 12 or 13, wherein an average particle diameter of the plurality of liquid droplets is 100 µm or more and 500 µm or less.

15. The cell detachment method according to any one of claims 12 to 14, wherein an average speed of the plurality of liquid droplets is 5 km/h or more and 50 km/h or less.

16. The cell detachment method according to any one of claims 12 to 15, wherein an average kinetic energy of the plurality of liquid droplets is 5.0 × 10⁻¹³ J or more and 6.3 × 10⁻⁹ J or less.

17. The cell detachment method according to any one of claims 12 to 16, wherein a total discharge amount of the plurality of liquid droplets by one discharge is 3.0 mL or less.

18. The cell detachment method according to any one of claims 12 to 17, wherein a discharge time of the plurality of liquid droplets by one discharge is 0.2 seconds or less.

19. The cell detachment method according to any one of claims 12 to 18, wherein a distance between the culture surface and the position is 10 mm or more and 50 mm or less.

20. The cell detachment method according to any one of claims 12 to 19, further comprising draining the liquid from the culture vessel to reduce an amount of the liquid in the culture vessel so that a portion of the surface of the cells that is in contact with air is expanded and the plurality of liquid droplets are discharged to the expanded portion in contact with air.
